(19) Europäisches Patentamt
European Patent Office
Office européen des brevets

(11) Publication number: 0 393 747
A2

(12) EUROPEAN PATENT APPLICATION

(21) Application number: 90200868.9

(22) Date of filing: 10.04.90

(51) Int. Cl.5: A61K 31/235, A61K 9/22, A61K 9/32, A61K 9/36, A61K 9/52

(30) Priority: 20.04.89 US 341338
27.02.90 US 483472

(43) Date of publication of application:
24.10.90 Bulletin 90/43

(84) Designated Contracting States:
AT BE CH DE DK ES FR GB GR IT LI LU NL SE

(71) Applicant: THE PROCTER & GAMBLE COMPANY
One Procter & Gamble Plaza
Cincinnati Ohio 45202(US)

(72) Inventor: Mandel, Kenneth Gary
5740 Auberger Drive
Fairfield, OH 45014(US)
Inventor: Sheldon, Russell James
93 Ridge Drive
Fairfield, OH 45014(US)

(74) Representative: Suslic, Lydia et al
Procter & Gamble European Technical Center N.V. Temselaan 100
B-1820 Strombeek-Bever(BE)

# (54) Mebeverine dosage form.

(57) The present invention involves pharmaceutical compositions for the peroral administration of mebeverine comprising a safe and effective amount of mebeverine, and a pharmaceutically-acceptable carrier which comprises means for attaining the transport of the mebeverine through the gastrointestinal tract of the patient to its lower part and means for releasing the mebeverine in the lumen of the lower gastrointestinal tract.

EP 0 393 747 A2

# MEBEVERINE DOSAGE FORM

## TECHNICAL FIELD

This invention is concerned with a novel dosage form and method of administering mebeverine in order to achieve superior antispasmodic activity of the drug in the lower gastrointestinal tract. More particularly, it is concerned with methods and means for dosing mebeverine locally to the lower gastrointestinal tract.

## BACKGROUND OF THE INVENTION

Mebeverine, 3,4-dimethoxybenzoic acid 4-(ethyl(2-(4-methoxyphenyl)-1-methylethyl)amino)butyl ester, is disclosed in the Merck Index, Tenth Edition (1983), M. Windholz, ed., No. 5590, page 821. The use of mebeverine for treatment of spasmodic conditions of the colon is disclosed in U.S. Patent No. 3,265,577 issued to Kralt, Moed, Asma & Lindner on August 9, 1966. Other references which disclose mebeverine and its uses include the following: Lindner, A., H. Selzer, V. Claassen, P. Gans, O.R. Offringa, & J.M.A. Zwagemakers, "Pharmacological Properties of Mebeverine, a Smooth-muscle Relaxant", Arch. Int. Pharmacodyn., Vol. 145 (1963), pp. 378-395; Connell, A.M., "Physiological and Clinical Assessment of the Effect of the Musculotopic Agent Mebeverine on the Human Colon", British Medical Journal, Vol. 2 (1985), pp. 848-851; Subissi, A., P. Brunori, & M. Bachi, "Effects of Spasmolytics on $K^+$-induced Contraction of Rat Intestine In Vivo", European Journal of Pharmacology, Vol. 96 (1983), pp. 295-301; Dureng, G., C. Boero, A. Meunier, & C. Labrid, "Puissance Relative de Quelques Antispasmodiques au Niveau du Tractus Digestif et de la Vessie, Chez le Chien Anesthesie", J. Pharmacol. (Paris), Vol. 12 (1981), pp. 135-145; Denavit, M., F. Achard, I. Morer, & M. Roche, "Reorganisation du Profil Moteur Digestif par la Mebeverine. Analyse Electromyographique chez la Chien", C. R. Soc. Biol., Vol. 177 (1983), pp. 626-631; Den Hertog, A., & J.V. den Akker, "Modification of α-receptor-operated Channels by Mebeverine in Smooth Muscle Cells of Guinea-pig Taenia Caeci", European Journal of Pharmacology, Vol. 138 (1987), pp. 367-374; Den Hertog, A., & J.V. den Akker, "The Action of Mebeverine and Metabolites on Mammalian Non-myelinated Nerve Fibres", European Journal of Pharmacology, Vol. 139 (1987), pp. 353-355. A controlled release dosage form of mebeverine is disclosed in Belgian Patent No. 887,186 of Ady Trenker, published July 22, 1981. A formulation of mebeverine designed for once daily administration by Elan is disclosed in Pharmaprojects, July, 1985, au 63 T1A.

Mebeverlne is generally taken in a peroral dosage form; it is readily absorbed from the upper gastrointestinal tract to provide a substantial systemic dose. Although the antispasmodic activity provided by such a systemic dose of mebeverine has long been known, mebeverine is not a universally accepted drug; for example, it has never been approved for use in the United States. One concern regarding mebeverine is that systemic doses of mebeverine have been known to decrease heart rate and blood pressure in animals and humans.

## SUMMARY OF THE INVENTION

It is an object of the present invention to provide a dosage form of mebeverine which delivers the mebeverine locally to the lower gastrointestinal tract.

It is a further object of the present invention to provide a dosage form of mebeverine which provides therapeutic antispasmodic activity to the lower gastrointestinal tract at substantially lower doses than is required with normal peroral dosage forms of mebeverlne.

It is also an object of the present invention to provide a method for treating spasmodic conditions of the lower gastrointestinal tract by local treatment with mebeverine.

It is a further object of the present invention to provide a method for achieving longer antispasmodic efficacy while maintaining lower systemic (blood) level of mebeverine than is normally experienced with peroral dosing of mebeverine for treatment of such spasmodic conditions.

It is also an object of the present invention to provide a method of treating such spasmodic conditions by achieving substantial antispasmodic efficacy without the occurrence of substantial antimotility activity.

The present invention involves pharmaceutical compositions in dosage unit form, for the peroral administration of mebeverine to a human or lower animal having a gastrointestinal tract with a lower part and a lumen therethrough, comprising:

(a) a safe and effective amount of mebeverine; and

(b) a pharmaceutically-acceptable carrier which comprises means for attaining the transport of said mebeverine through said gastrointestinal tract to said lower part after ingestion of said composition, and means for attaining the release of an effective amount of said mebeverine in said lumen of said lower part of the gastrointestinal tract.

## DETAILED DESCRIPTION OF THE INVENTION

As used herein, "lower part of the gastrointestinal tract" or "lower gastrointestinal tract" means the distal small intestine and the colon. As used herein, "distal small intestine" means the ileum. As used herein, "upper gastrointestinal tract" means the esophagus, the stomach and the proximal small intestine. As used herein, "proximal small intestine" means the duodenum and the jejunum.

As used herein, "attaining the transport of ... mebeverine through the gastrointestinal tract" means retaining the mebeverine in the gastrointestinal tract at least until it reaches the specified part of the gastrointestinal tract. Because mebeverine is readily absorbed from the gastrointestinal tract, especially from the duodenum, into the systemic system, the mebeverine must be protected from such absorption in some manner, if its transport is to be attained along a substantial portion of the gastrointestinal tract, particularly to the lower gastrointestinal tract.

As used herein, "attaining the release of ... mebeverine" means releasing the mebeverine from any substantial constraint and making it available for contact with the intestinal juices and lumenal surfaces in the gastrointestinal tract.

As used herein, "attaining the delivery of ... mebeverine locally to the lumen of the lower gastrointestinal tract" means that the mebeverlne is transported to and is released in the lumen of the lower gastrointestinal tract, such that the mebeverine contacts the lumenal surfaces of the lower gastrointestinal tract.

Mebeverine is known to have pharmacological activity as an antispasmodic agent. As used herein, "mebeverine" means 3,4-dimethoxybenzoic acid 4-(ethyl(2-(4-methoxyphenyl)-1-methylethyl)amino)butyl ester:

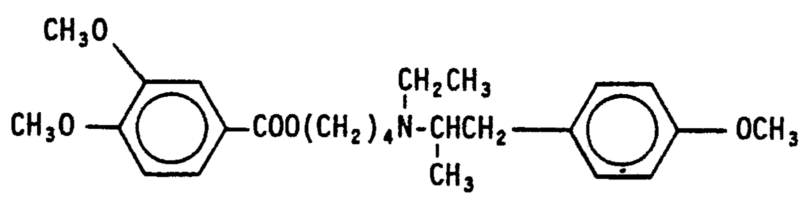

and the pharmaceutically-acceptable salts thereof. A preferred form of mebeverine is the hydrochloride salt of addition. Other pharmaceutically-acceptable salts such as the phosphate, bromide, acetate, tartrate and benzoate are also known.

The present invention involves compositions of mebeverine useful for treating or preventing spasmodic conditions of the lower gastrointestinal tract. Such spasmodic conditions may be induced by any of a number of different stimuli, including food, stress, and colonic distention. Spasmodic conditions of the lower gastrointestinal tract which can be effectively treated or prevented using the compositions of the present invention include functional intestinal/colonic disorders, such as Functional Bowel or Irritable Bowel Syndrome (IBS). IBS is also commonly known as, and includes: irritable colon and its syndrome, spastic colon, spastic colitis, mucous colitis, and spastic bowel. The compositions of the present invention are also expected to be effective for treating certain cases of diarrhea and/or constipation.

IBS is currently believed to be a functional motor disorder of the gastrointestinal tract. Clinical symptoms which can be treated or prevented by administration of the compositions of the present invention include abdominal pain and/or altered bowel habit (diarrhea or constipation). The following references provide information regarding IBS: Krag, E., "Irritable Bowel Syndrome: Current Concepts and Future Trends", Scandanavian Journal of Gastroenterology, Vol. 20 (supplement 109) (1985), pp. 107-115; Thompson, W.G., "The Irritable Bowel", Gut, Vol. 25 (1984), pp. 305-332; Drossman, D.A., R.S. Sandler, D.C. McKee & A.J. Lovitz, "Bowel Patterns Among Subjects Not Seeking Health Care - Use of a

Questionnaire to Identify a Population with Bowel Dysfunction:, Gastroenterology, Vol. 83 (1982), pp. 529-534; Sandler, R.S., D.A. Drossman, H.P. Nathan & D.C. McKee, "Symptom Complaints and Health Care Seeking Behavior in Subjecis with Bowel Dysfunction", Gastroenterology, Vol. 87 (1982), pp. 314-318; Drossman, D.A. & B.C. Lowman, "Irritable Bowel Syndrome: Epidemiology, Diagnosis and Treatment", Clinical Gastroenterology, Vol. 14 (1985), pp. 559-573; Snape, W.J., Jr., G.M. Carlson, S.A. Matarazzo & S. Cohen, "Evidence That Abnormal Myoelectrical Activity Produces Colonic Motor Dysfunction in the Irritable Bowel Syndrome", Gastroenterology, Vol. 72 (1977), pp. 383-387; Kumar, D. & D.L. Wingate, "The Irritable Bowel Syndrome: A Paroxysmal Modor Disorder", Lancet, November 2, 1985, pp. 973-977; Kellow, J.E. & S.F. Phillips, "Small Bowel Dysmotility Correlates with Symptoms in Irritable Bowel Syndrome", Gastroenterology, Vol. 90 (1986), p. 1488 (abstract); Kellow, J.E., R.C. Gill & D.L. Wingate, "Proximal Gut Motor Activity in Irritable Bowel Syndrome (IBS) Patients at Home and at Work", Gastroenterology, Vol. 92 (1987), p. 1463 (abstract); Kellow, J.E. & S.F. Phillips, "Altered Small Bowel Motility in Irritable Bowel Syndrome is Correlated with Symptoms", Gastroenterology, Vol. 92 (1987), pp. 1885-1893.

The present invention involves the surprising discovery that mebeverine, when delivered locally to the lumen of the lower gastrointestinal tract, provides greater antispasmodic activity compared to that achieved with normal peroral doses of mebeverine which are absorbed into the systemic system from the stomach and/or upper gastrointestinal tract. This greater antispasmodic activity may be manifested as a lessening of frequency or severity of spasmodic episodes. Therefore, one aspect of the present invention is a method for treating functional intestinal/colonic disorders, of a human or lower animal, having a lower gastrointestinal tract with a lumen therethrough, by attaining the delivery of a safe and effective amount of mebeverine locally to said lumen of the lower gastrointestinal tract of said human or lower animal. Other known antispasmodic agents often do not exhibit such greater antispasmodic activity when dosed to the lumen of the lower gastrointestinal tract in this manner, e.g., dicyclomine HCl. Once the mebeverine is in contact with the lumenal surfaces of the lower gastrointestinal tract, it may be locally absorbed into the tissues thereof.

Mebeverine has been found to not only provide good antispasmodic activity when delivered locally to the lumen of the lower gastrointestinal tract, but it has been found to provide such therapeutic activity at a substantially lower dose compared to that required to achieve therapeutic activity from a normal peroral dose. By using lower therapeutic doses of mebeverine, systemic absorption of the mebeverine can be substantially reduced; thus reducing the risk of the occurrence of side effects such as decreased heart rate and blood pressure.

Normal contractions in the walls of the colon are desirable in order to achieve basal propulsive motility of materials through the colon. A drug which substantially affects such normal contractions of the colon will reduce the basal motility in the colon; such activity is referred to herein as "antimotility" activity of a drug. Those suffering from spasmodic conditions of the colon, such as IBS and other disorders disclosed hereinabove, experience spasmodic episodes of the colon in addition to the normal contractions which provide basal motility. Such spasmodic episodes can cause pain and can prevent the normal movement of material through the colon. A drug which prevents or reduces such spasmodic episodes is referred to herein as having "antispasmodic" activity.

Mebeverine, when dosed systemically (e.g., intravenously or perorally such that the mebeverine is absorbed into the blood from the stomach and/or proximal small intestine), has similar antimotility and antispasmodic activity at a given dosage level. Therefore, a systemic dose of mebeverine which is effective for reducing spasmodic episodes of IBS and other colonic disorders also reduces basal motility in the colon, an undesirable side effect. In contrast, when mebeverine is dosed locally to the lumen of the colon according to the methods of the present invention, it provides effective antispasmodic activity at a substantially lower dose than is required to produce undesired antimotility activity.

A preferred embodiment of the present invention is a method for treating functional colonic disorders, especially IBS, of a human or lower animal having a colon with a lumen therethrough, by attaining the delivery of mebeverine locally to said lumen of the colon in an amount such that substantial antispasmodic activity is achieved without the occurrence of substantial antimotility activity. Preferred methods of the present invention attain the local delivery of mebeverine to the lumen of the lower gastrointestinal tract, preferably of the colon, of from about 0.01 mg/kg to about 30 mg/kg, preferably from about 0.1 mg/kg to about 10 mg/kg, per dose. More preferred methods of the present invention attain such local delivery of mebeverine of from about 0.3 mg/kg to about 1 mg/kg or from about 1 mg/kg to about 3 mg/kg per dose. Such doses of mebeverine are preferably delivered locally to the lumen and released therein as a bolus dose, or more preferably released therein intermittently or continually over a period of up to about 1 hr, or more preferably up to about 12 hrs. More preferred are such doses which attain a controlled local release of the mebeverine to the lumen over a period of from about 1 hr to about 2 hrs or to about 8 hrs, still more preferably of from about 2 hrs to about 4 hrs or to about 6 hrs.

Preferred methods of the present invention attain local delivery and release of mebeverine to the lumen of the lower gastrointestinal tract, preferably of the colon, intermittently or continually over a sustained period at an average rate of from about 0.1 mg/hr to about 70 mg/hr, preferably from about 0.3 mg/hr to about 35 mg/hr, more preferably from about 1 mg/hr to about 3 mg/hr or from about 3 mg/hr to about 10 mg/hr, over the above periods. Compositions capable of providing such rates of local delivery and release of mebeverine to the lumen of the lower gastrointestinal tract are described hereafter.

Another aspect of the present invention involves pharmaceutical compositions in dosage unit form, for the peroral administration of mebeverine to a human or lower animal having a gastrointestinal tract with a lower part and a lumen therethrough, comprising:

(a) a safe and effective amount of mebeverine; and

(b) a pharmaceutically-acceptable carrier which comprises means for attaining the transport of said mebeverine through said gastrointestinal tract to said lower part after ingestion of said composition, and means for attaining the release of an effective amount of said mebeverine in said lumen of said lower part of the gastrointestinal tract.

The methods and compositions of the present invention comprise a safe and effective amount of mebeverine. The phrase "safe and effective amount", as used herein, means an amount of mebeverine high enough to provide a significant positive modification of the condition to be treated, but low enough to avoid serious side effects (at a reasonable benefit/risk ratio), within the scope of sound medical judgement. A safe and effective amount of mebeverine will vary with the particular condition being treated, the age and physical condition of the patient being treated, the severity of the condition, the duration of the treatment, the nature of concurrent therapy and like factors. As indicated hereinabove, an effective dose of mebeverine in compositions of the present invention is preferably substantially lower than the dose of mebeverine required to achieve efficacy via normal peroral dosing.

A safe and effective dose of mebeverine in a composition of the present invention provides from about 10 mg/day to about 2000 mg/day of mebeverine locally to the surface of the lumen of the lower gastrointestinal tract of a human patient. A more preferred amount of mebeverine dosed to a human patient is from about 20 mg/day to about 1200 mg/day; more preferred still is from about 40 mg/day to about 800 mg/day; still more preferred is from about 70 mg/day to about 400 mg/day; also more preferred is from about 100 mg/day to about 200 mg/day, or from about 200 mg/day to about 600 mg/day. It is preferred that the mebeverine be provided to a patient such that the mebeverine is present in the lumen of the lower gastrointestinal tract of the patient for an extended period of time. This can be achieved by multiple dosing of proportional quantities of the above amounts throughout a day, or by the use of time-release dosage unit forms.

As used herein, the term "pharmaceutically-acceptable carrier" denotes a compatible solid and/or liquid filler, diluent, and/or encapsulating substance. By "compatible" as used herein, it is meant that the components are capable of being commingled without interacting in a manner which would substantially decrease the pharmaceutical efficacy of the mebeverine under ordinary use situations.

Because mebeverine is readily absorbed into the systemic circulation from the upper gastrointestinal tract, an objective of a preferred dosage unit form composition of the present invention is to delay the release of mebeverine from the dosage form (and hence its availability for absorption) until the dosage form has emptied from the stomach and preferably transited a substantial portion of the proximal small intestine. It is preferable that the release of substantially all the mebeverine of the dosage unit form is so delayed. An additional objective of a more preferred dosage unit form of the present invention is to control the subsequent rate of release of mebeverine in a manner such that maximum local pharmacological activity is achieved.

A preferred dosage unit form of a composition of the present invention comprises two functional parts: (1) a core composition that contains the mebeverine and controls its rate of release; and (2) a targeting composition that delays the release of mebeverine from the core composition until after the dosage form has emptied from the stomach. It is preferable that the targeting composition delays the release of the mebeverine from the core composition until after the dosage form has transited a portion of the small intestine, preferably past the duodenum, more preferably past the jejunum also, sometimes also past the ileum. Typically, the core composition is encased within the targeting composition, such that the targeting composition acts as a shell, or coating surrounding the core composition. In certain executions, the targeting composition may also act to control the rate of mebeverine release in conjunction with the core composition.

Core Composition

A preferred core composition of the present invention preferably physically exists as a finely dispersed powder, nonaqueous liquid or semi-liquid solution or suspension, pellet, compressed or molded tablet, or hard or soft gelatin capsule containing one or more of the preceding physical forms. The core composition may incorporate one or more excipients to improve physical integrity, increase stability, increase ease of manufacture, or alter the rate of mebeverine release. These physical forms of the core composition are further described hereinbelow.

(1) Dispersed Powder:

The core composition may comprise a finely dispersed powder (particle size typically less than about 0.1 mm) of mebeverine that is incorporated into a matrix of the targeting composition, or encapsulated in a hard gelatin capsule that is subsequently coated with the targeting composition. The finely dispersed mebeverine powder core composition may also include various excipients such as diluents (e.g., lactose, sucrose, starch, calcium sulfate, dicalcium phosphate, microcrystalline cellulose); binders (e.g., polyvinylpyrrolidone, pregelatinized starch, gelatin, hydroxypropyl methylcellulose, methylcellulose, hydroxyethyl cellulose, hydroxypropyl cellulose); lubricants (e.g., stearic acid, magnesium stearate); disintegrants (e.g., sodium starch glycolate, cross-linked polyvinylpyrrolidone, cross-linked carboxymethyl cellulose); glidants (e.g., fumed silica); and buffers. In addition, excipients designed to retard the release rate of mebeverine, such as water insoluble polymers (e.g., ethyl cellulose, polymethacrylates (e.g., Eudragit types RL, RS, and E30D, Rohm Tech, Inc. Darmstadt, West Germany), pH sensitive (enteric) polymers (polymethacrylates (e.g., Eudragit types L, S, L30D, Rohm Pharma, Darmstadt, West Germany), cellulose acetate phthalate, hydroxypropyl methylcellulose phthalate, shellac, polyvinyl acetate phthalate), and water insoluble solid lipids (e.g., tristearin), may be incorporated into the mebeverine powder. The powder may be prepared via a number of techniques well-known to pharmaceutical science such as dry mixing, wet granulation, and fluid bed granulation.

(2) Non-Aqueous Solution or Dispersion:

The core composition may comprise a solution or dispersion of mebeverine in a non-aqueous vehicle. These compositions are typically encapsulated in hard or soft gelatin capsules which are subsequently coated with the targeting composition. The vehicle may be liquid or semi-liquid in form. It may consist of polyols (e.g., polyethylene glycol, propylene glycol, glycerine), nonionic surfactants (e.g., polysorbates, poloxamers), or lipids (e.g., fixed oils, medium chain triglycerides, fatty acids). Certain vehicles may be employed to retard the release of mebeverine from the solution/dispersion core composition. These include water insoluble lipids and polymers that form viscous gels when contacted with an aqueous medium (e.g., certain poloxamers). In addition, certain polyols may be thickened with cellulosic polymeric derivatives (e.g., hydroxyethyl cellulose) and thus retard the release of mebeverine. Such solutions/dispersions may be prepared by dissolving or dispersing mebeverine in the vehicle with mild heating if necessary.

(3) Pellets:

The core composition may comprise a mebeverine pellet (typical particle size of from about 0.1 mm to about 4 mm, preferably from about 1 mm to about 2 mm). The pellets may be individually coated with or incorporated into a matrix of the targeting composition, or they may be filled into a hard gelatin capsule which is subsequently coated with the targeting composition. The pellets may be prepared via dry granulation (roller compaction), slugging, or coating an inert or active substrate (includes powder coating and solution/suspension coating). Pellet core compositions may include those excipients listed for finely dispersed powder core compositions that are appropriate for the preparation process employed.

(4) Compressed Tablets:

The core composition may comprise a compressed mebeverine tablet. The compressed tablet may comprise a rapidly disintegrating matrix, a slowly erodible matrix, or a non-erodible matrix. A rapidly disintegrating matrix is intended to release mebeverine over a relatively short period of time. It may include

any of the excipients listed for finely dispersed powder core compositions. Slowly erodible and non-erodible matrices are intended to retard the release of mebeverine. Mebeverine is released from slowly erodible matrices via a combination of surface dissolution of the matrix component(s) and diffusion of mebeverine through the undissolved matrix. Slowly erodible matrix components include cellulose derivatives (e.g., methylcellulose, carboxymethyl cellulose, hydroxyethyl cellulose, hydroxypropyl cellulose), and enteric polymers. Mebeverine is released from non-erodible matrices via diffusion of mebeverine through the matrix. Examples of non-erodible matrix components include polymethacrylates, ethyl cellulose and solid lipids. Compressed tablet core compositions may be prepared, for example, via direct compression, and via wet granulation, dry granulation, or fluid bed granulation followed by compression.

(5) Molded Tablets:

The core composition may comprise a molded mebeverine tablet. Molded tablets consist of a solution or dispersion of mebeverine in a solid matrix. The matrix excipients are such that they may be melted at a temperature that will not degrade mebeverine and include, for example, polyethylene glycols, poloxamers, and various lipids. Those exciplents that are water insoluble, or that form viscous gels when contacted with an aqueous medium will reduce the rate of mebeverine release. Molded tablets are prepared, for example, by melting excipients, dissolving or dispersing mebeverine in the melt, pouring the melt into tablet molds, and allowing the melt to solidify.

It is preferred that the core compositions of the present invention retard the rate of mebeverine release such that increased portions of the gastrointestinal tract (distal to the point at which the targeting composition permits the initiation of mebeverine release from the core composition) are exposed to mebeverine as the core composition transits the lumen of the gastrointestinal tract.

Targeting Composition

A preferred targeting composition of the present invention typically encases the core composition and acts to delay the release of mebeverine until the dosage unit form has emptied from the stomach and, preferably transited a portion of the small intestine. The targeting composition preferably exists as a matrix into which the core composition (finely dispersed powder, pellets) is incorporated, or as a coating over the core composition (individual pellets, compressed or molded tablets, hard or soft gelatin capsules). The targeting composition is preferably comprised of (1) an enteric material that will not dissolve in the gastric environment, but will dissolve/disperse at some point after the dosage unit form has emptied from the stomach; or (2) a combination of such an enteric material in an outer layer and an inner layer comprised of a timed-release material that will dissolve slowly at a rate independent of the intestinal lumenal environment. Regarding (1) of this paragraph, the enteric material both prevents the release of mebeverine in the stomach and controls the site in the small intestine/colon at which the release of mebeverine is initiated based upon the environmental factors affecting its dissolution and the thickness of the enteric layer. Regarding (2) of this paragraph, the enteric outer layer primarily prevents release of mebeverine in the stomach, and the timed-release material controls the site in the small intestine/colon at which the release of mebeverine is accomplished based upon its dissolution rate and thickness, and intestinal transit rate.

There are four general preferred types of targeting compositions: (1) timed-release matrix overcoated with an enteric layer; (2) enteric matrix; (3) timed-release coat overcoated with an enteric coat; and (4) enteric coat. Finely dispersed powder and pellet core compositions are preferred for use with matrix targeting compositions, whereas individual pellets, compressed or molded tablets, and hard or soft gelatin capsules are preferred core compositions for use with coated targeting compositions.

The matrix targeting compositions are preferably prepared via blending the matrix material (either enteric or timed-release) with the core composition and other appropriate tableting excipients, followed by compression into tablets. If a timed-release matrix is employed, the resulting matrix tablet is subsequently overcoated with a suitable enteric material as described below.

The coated targeting compositions are preferably prepared via coating the core composition with either an enteric material, or a timed-release material followed by an enteric material. Coating aids such as plasticizers and talc may be incorporated into the coated targeting composition. Pellets, molded and compressed tablets, and soft and hard gelatin capsules are typically coated in fluidized bed equipment, tablets and capsules are typically coated in perforated pans, and tablets may also be coated via compression coating.

Individually coated pellets are typically encapsulated in hard gelatin capsules to provide a finished dosage unit form.

Preferred timed-release materials include cellulosic derivatives, such as methyl cellulose, carboxymethyl cellulose, hydroxyethyl cellulose, hydroxypropyl cellulose, and hydroxypropyl methylcellulose. The dissolution rate of these and other timed-release materials is largely pH independent and will be a function of molecular weight and degree of substituent substitution. In addition, in matrix targeting compositions, the ratio of the timed-release material to core composition, type and level of tableting excipients, and compression pressure may affect the rate of dissolution. For coated targeting compositions, the thickness of the layer of timed-release material, coating conditions, and type and level of coating aids may influence the rate of dissolution. The rate of dissolution of the timed-release material in combination with the intestinal transit rate of the dosage form will control the site in the intestinal lumen at which mebeverine release is initiated.

Preferred enteric materials include pH-sensitive polymers, such as-polymethacrylates (e.g., Eudragit Types L, S, L-55, Rohm Pharma, Darmstadt, West Germany), cellulose acetate phthalate, hydroxypropyl methylcellulose phthalate, shellac, polyvinyl acetate phthalate, which are insoluble at the pH of the gastric environment, but will dissolve at various pH's above about pH 5. The pH at which the pH-sensitive polymer begins to dissolve and the amount of polymer (ratio of polymer to core composition in matrix systems, thickness of coat in coated systems) will primarily influence the site in the intestinal lumen at which mebeverine release is initiated. Typically, higher pH dissolution points and increased amounts of pH-sensitive polymer will increase the distance the dosage form will travel in the small intestine/colon prior to release of mebeverine. For compositions of the present invention, preferred pH-sensitive enteric materials dissolve only at a pH of greater than about 5.5; more preferred enteric materials dissolve only at pH of greater than about 6; also more preferred at pH of greater than about 6.5; also preferred are enteric materials which dissolve only at a pH of greater than about 7.

Other preferred enteric materials include water insoluble lipids that resist dissolution in the stomach, but are solubilized by bile salts and/or hydrolyzed by intestinal enzymes in the small intestine.

Coated enteric targeting compositions are preferred, with a pH-sensitive enteric material for such targeting compositions preferred. Especially preferred is a polymethacrylate polymer (Eudragit S) with a pH dissolution value of about pH 7. A dosage form consisting of individually enteric coated pellets encapsulated in a hard gelatin capsule is also especially preferred as the gastric emptying of this type of dosage form is not appreciably affected by the fed state.

The following non-limiting examples provide typical formulations for compositions of the present invention, and typical methods for treating human disorders with such compositions.

## EXAMPLE 1

## SUSTAINED RELEASE, ENTERIC COATED PELLETS

### Core

The core of the pellets contains 80% mebeverine, and 20% acrylic methacrlylic acid ester copolymer (Eudragit RS 30 D, Rohm Pharma).

### Enteric coating

The enteric coating for the pellets contains 22% methacrylic acid methylmethacrylate copolymer (Eudragit L 100, Rohm Pharma), 22% methacrylic acid methylmethacrylate copolymer (Eudragit S 100, Rohm Pharma), 40% ethylacrylate methylmethacrylate copolymer (Eudragit NE 30 D, Rohm Pharma), 15% talc (Talc USP, Penta Manufacturing Company), and 1% hydroxypropylmethyl cellulose (Methocel, Dow Chemical Company).

Method of manufacture

One kilogram of mebeverine cores are prepared in a rotary fluidized-bed granulator. Spheronization is performed by spraying Eudragit RS 30 D onto the fluidized mebeverine at an average rate of 100 ml/min for 7 minutes with an atomization pressure of 3 bar. The air inlet temperature is maintained at 70° C during the spheronization and drying.

The 20% enteric coating solution is prepared by combining a 30% aqueous suspension of Eudragit L 100 in 1.1% NaOH, a 30% aqueous suspension of Eudragit S 100 in 0.7% NaOH, and a 30% aqueous suspension of Eudragit NE 30 D with the talc dispersed in an aqueous hydroxypropylmethyl cellulose solution.

The coating is applied in a Wurster fluidized bed coater (UniGlatt, Glatt Air Techniques) at a spray rate of 4 ml/min at an atomization pressure of 2 bar. The air inlet temperature is maintained at 60° C. The coating is applied at a level of 15% of the total pellet weight.

The above enteric coating begins to dissolve at an appreciable rate at a pH of about 6.5.

The coated pellets are filled into No. 2 hard gelatin capsule shells, such that each capsule contains about 100 mg of mebeverine.

## EXAMPLE 2

## STANDARD RELEASE, ENTERIC COATED PELLETS

Core

The core of the pellets contains 80% mebeverine and 20% microcrystalline cellulose (Avicel, FMC).

Enteric coating

The enteric coating for the pellets contains 75% methacrylic acid methylmethacrylate copolymer (Eudragit L 30 D, Rohm Pharma), 7% polyethylene glycol (E-6000, BASF Wyandotte Corp.), and 18% talc (Talc USP, Penta).

Method of manufacture

One kilogram of mebeverine cores are prepared by premixing mebeverine and microcrystalline cellulose in a rotary fluidized-bed granulator. Spheronization is performed by spraying distilled water at an average rate of 100 ml/min for 10 minutes with an atomization pressure of 3 bar. The air inlet temperature is maintained at 70° C during the spheronization and drying.

The 22% enteric coating solution is prepared by combining the 30% aqueous suspension of Eudragit L 30 D with the PEG 6000 solution into which the talc has been homogenized.

The coating is applied in a Wurster fluidized bed coater at a spray rate of 4 ml/min at an atomization pressure of 2 bar. The air inlet temperature is maintained at 60° C. The coating is applied at a level of 25% of the total pellet weight.

The above enteric coating begins to dissolve at an appreciable rate at a pH of about 5.5.

The coated pellets are filled into No. 2 hard gelatin capsule shells, such that each capsule contains about 100 mg of mebeverine.

## EXAMPLE 3

SUSTAINED RELEASE, ENTERIC COATED TABLETS

Core

The core of the tablets contains 20% mebeverine, 59% lactose (Fast Flo Lactose, Foremost Whey Products), 20% acrylic methacrlylic acid ester copolymer (Eudragit RS PM, Rohm Pharma), 0.5% magnesium stearate (Mallinckrodt, Inc.), and 0.5% colloidal silica (Cab-O-Sil, Cabot Corporation).

Enteric coating

The enteric coating for the tablets contains 68% methacrylic acid methylmethacrylate copolymer (Eudragit S 100, Rohm Pharma), 14% dibutyl phthalate, and 18% talc (Talc USP, Penta).

Method of manufacture

Mebeverine cores are prepared by mixing a 2:1:1 mixture of the mebeverine with lactose and Eudragit RS PM in a forced mixer for 5 minutes. After this mixing time, a 1:1 mixture of ethanol and distilled water is added to the dry mixture and mixing is continued for 5 minutes. The wet mass is granulated through a 40 mesh sieve and dried for 12 hours at 40° C. The dry granulate is passed through a 40 mesh sieve.

The granulated mebeverine is combined with the remainder of the lactose and Eudragit RS PM and mixed in a tumbler mixer for 30 minutes. The magnesium stearate and colloidal silica are then added and mixing is continued for 10 minutes. The resulting tablet blend is directly compressed into 500 mg cores.

The 13% enteric coating solution is prepared by combining a 20% Eudragit S 100 in a 9:2 isopropyl alcohol:acetone solution with talc suspended in 9:2 isopropyl alcohol:acetone, dibutyl phthalate solution.

The coating is applied in a pan coater at a rate of 8 ml/min through a 1.0 mm nozzle having an atomization pressure of 3 bar. The inlet air temperature is maintained at 45° C. The coating is applied at a level of 15% of the total tablet weight.

The above enteric coating begins to dissolve at an appreciable rate at a pH of about 7.0.

EXAMPLE 4

STANDARD RELEASE, ENTERIC COATED TABLETS

Core

The core of the tablets contains 20% mebeverine, 59% lactose (Fast Flo Lactose, Foremost), 20% microcrystalline cellulose (Avicel, FMC Corp.), 0.5% magnesium stearate (Mallinckrodt), and 0.5% colloidal silica (Cab-O-Sil, Cabot).

Enteric coating

The enteric coating for the tablets contains 22% methacrylic acid methylmethacrylate copolymer (Eudragit S 100, Rohm Pharma), 40% acid methylmethacrylate copolymer (Eudragit S 100, Rohm Pharma), 40% ethylacrylate methylmethacrylate copolymer (Eudraget NE 30 D, Rohm Pharma), 15% talc (Talc USP, Penta), and 1% hydroxypropylmethyl cellulose (Methocel, Dow Chemical Company).

The mebeverine is granulated and is combined with the remainder of the lactose and microcrystalline cellulose and mixed in a tumbler mixer for 30 minutes. The magnesium stearate and colloidal silica are then added and mixing is continued for 10 minutes. The resulting tablet blend is directly compressed into 500

mg cores.

The 20% enteric coating solution is prepared by combining a 30% aqueous suspension of Eudragit L 100 in 1.1% NaOH, a 30% aqueous suspension of Eudragit S 100 in 0.7% NaOH, and a 30% aqueous suspension of Eudragit NE 30 D with the talc dispersed in an aqueous hydroxypropylmethyl cellulose solution.

The coating is applied in a pan coater at a rate of 7 ml/min through a 1.0 mm nozzle having an atomization pressure of 3 bar. The inlet air temperature is maintained at 80 °C. The coating is applied at a level of 15% of the total tablet weight.

The above enteric coating begins to dissolve at an appreciable rate at a pH of about 6.5.

## EXAMPLE 5

A person suffering with abdominal pain and cramps is diagnosed as having IBS. The person ingests 1 capsule of Example 1 hereinabove each day at bedtime for 5 days. The pain and cramps greatly diminish during the first day after dosing and completely subside after three days of dosing.

## EXAMPLE 6

A person suffering with abdominal pain is diagnosed as having IBS. The person ingests a tablet of Example 4 hereinabove every 12 hours for three days. The pain subsides within 12 hours of the initiation of dosing.

## EXAMPLE 7

A person suffering with abdominal pain is diagnosed as having IBS. The person ingests a tablet of Example 3 hereinabove after breakfast and lunch and before bedtime, for seven days. The pain subsides after one day of dosing.

While particular embodiments of the present invention have been described, it will be obvious to those skilled in the art that various changes and modifications of the present invention can be made without departing from the spirit of the scope of the invention. It is intended to cover, in the appended claims, all such modifications that are within the scope of this invention.

## Claims

1. A pharmaceutical composition in dosage unit form, for the peroral administration of mebeverine to a human or lower animal having a gastrointestinal tract with a lower part and a lumen therethrough, characterized in that it comprises:

(a) a safe and effective amount of mebeverine, preferably mebeverine hydrochloride, and

(b) a pharmaceutically-acceptable carrier which comprises means for attaining the transport of said mebeverine through said gastrointestinal tract to said lower part after ingestion of said composition, and means for attaining the release of an effective amount of said mebeverine in said lumen of said lower part of the gastrointestinal tract.

2. The composition of Claim 1 characterized in that said composition comprises:

(1) a core composition, preferably in the form of a dispersed powder, a pellet, or a compressed tablet, that contains the mebeverine and controls the rate of release of the mebeverine; and

(2) a targeting composition that delays the release of substantially all of the mebeverine from said core composition until after the dosage unit form has emptied from the stomach;

said core composition preferably being encased within the targeting composition.

3. The composition of Claim 2 characterized in that said targeting composition, preferably a pH-sensitive enteric material, delays the release of the mebeverine from said core composition until after the dosage unit form has passed the jejunum, said core composition preferably providing a sustained release of

the mebeverine.

4. The use of mebeverine, preferably mebeverine hydrochloride, for the manufacture of a medicament for treating functional intestinal/colonic disorders, especially Irritable Bowel Syndrome, of a human or lower animal having a lower gastrointestinal tract with a lumen therethrough, by attaining the delivery of a safe and effective amount of mebeverine locally to said lumen of the lower gastrointestinal tract of said human or lower animal.

5. The use of Claim 4 characterized in that said mebeverine is ingested perorally, by a human, preferably in an amount of from 20 mg/day to 200 mg/day, or from 200 mg/day to 600 mg/day, more preferably from 100 mg/day to 400 mg/day.

6. The use of mebeverine, preferably mebeverine hydrochloride, for the manufacture of medicament for treating functional intestinal/colonic disorders, especially Irritable Bowel Syndrome, of a human or lower animal having a colon with a lumen therethrough by attaining the delivery of mebeverine locally to said lumen of the colon in an amount such that substantial antispasmodic activity is achieved without the occurrence of substantial antimotility activity.

7. The use of Claim 6 characterized in that from 0.01 mg/kg to 30 mg/kg of mebeverine is dosed locally to the lumen of the colon over a period of from 0 hrs to 12 hrs.

8. The use of Claim 7 characterized in that from 0.1 mg/kg to 1 mg/kg of mebeverine is dosed locally to the lumen of the colon over a period of from 1 hr to 4 hrs, or from 1 mg/kg to 10 mg/kg of mebeverine is dosed locally to the lumen of the colon over a period of from 2 hrs to 8 hrs, preferably from 0.3 mg/kg to 3 mg/kg of mebeverine is dosed locally to the lumen of the colon over a period of from 1 hr to 6 hrs.